# EUROPEAN PATENT APPLICATION

(11) **EP 1 977 747 A1**
(43) Date of publication of application: **08.10.2008**
(21) Application number: 07090096.4
(22) Date of filing: 09.05.2007
(51) Int. Cl.: A61K 31/203, A61K 31/405, A61K 31/415, A61K 31/711, A61K 38/17, A61K 39/395

(54) **Adaptive cardiac remodeling by beta-catenin downregulation**

(30) Priority: 05.04.2007 EP 07090071
(71) Applicant: Max-Delbrück-Centrum, 13125 Berlin (DE)
(72) Inventor: Bergmann, Martin W., 13187 Berlin (DE); Hülsken, Jörg, 1066 Epalinges (CH); Birchmeier, Walter, 16341 Schwanebeck (DE); Taketo, Makoto Mark, Sakyo Kyoto 606-0815 (JP)
(74) Representative: Klages, Christlieb

(57) **Abstract**

The present invention relates to the use of an agent for downregulating or inhibiting β-catenin dependent signaling for the treatment of cardiovascular diseases or disorders,. as the specific inhibition of β-catenin dependent signaling by downregulating or inhibiting the expression or activity of β-catenin results in adaptive cardiac hypertrophy that is protective for example after angiotensin-II-induced stress.

## Description

### Background of the invention

Beta-catenin (β-catenin) is a plakophilin protein family member. The plakophilins belong to the armadillo-related proteins, which are essential components of the desmosomal plaque. In addition to there adhesive function, the plakophilin β-catenin has been ascribed an important signaling function. For instance, β-catenin is a transcriptional co-activator of the T cell factor/lymphoid enhancer factor (TCF/LEF) complex that regulates embryonic, postnatal, and oncogenic growth in many tissues, including the heart (Brembeck et al. Curr Opin Genet Dev. 2006;16:51-59).

Cardiomyocyte growth occurs during left ventricular (LV) remodeling following chronic pressure overload and/or ischemic heart disease. Increased β-catenin levels were detected in the intercalated disc in heart specimens from patients with inherited cardiac hypertrophy (Masuelli et al. Cardiovasc Res. 2003;60:376-387). The β-catenin increase at the cell membrane was accompanied by reduced nuclear β-catenin levels. Recently, reduced β-catenin - dependent transcription via Tcf/Lefl transcription factors was suggested to be the molecular mechanism behind the fibroadipocytic replacement in arrhytmogenic right ventricular cardiomyopathy caused by the loss of desmoplakin (Garcia-Gras et al. J Clin Invest. 2006;116:2012-2021).

LV remodeling includes re-activation of the fetal gene program and possibly the embryonic gene program as well (Liang et al. J Mol Cell Cardiol. 2002;34:611-616). WNT/β-catenin regulation is one of the earliest events in cardiac development. Specifically, β-catenin downregulation following inhibitory Dickkopf signaling in the embryonic endoderm precedes cardiac development from the embryonic mesoderm (Lickert et al. Dev Cell. 2002;3:171-181). Since universal β-catenin deletion is lethal, inducible tissue-specific modulation of β-catenin has been employed to analyze the role of this factor in adult heart left ventricular remodeling.

Two recent publications have analyzed the effect of β-catenin depletion in the adult heart: Zhou et al. (Am J Physiol Heart Circ Physiol. 2007;38:120) found no phenotype concerning membrane function due to the compensatory upregulation of plakoglobin in the intercalated disc as precedingly described in other tissues (Huelsken et al. Cell. 2001;105:533-545). Furthermore, Chen et al. (Mol. Cell. Biol. 2006;26:4462-4473) confirmed their preceding *in vitro* observations concerning β-catenin in adult cardiac hypertrophy: the increase in heart weight/body weight after trans-aortic constriction was attenuated in β-catenin depleted transgenic mice.

The WO 2004/094610 A2 discloses a system, method and compositions related to cardiomyocyte differentiation from a non-cardiomyocyte cell using factors that activate WNT/ β-catenin-signaling. The invention according to the WO 2004/094610 A2 relates to a cell-therapy system, wherein in a cell the WNT/ β-catenin signaling pathway is activated and this cell or a cell tissue resulting from this cell is used for the therapy of cardiac failure.

The report of Barandon et al. (Circulation 2003; 108:2282-2289) investigates the role of the protein Frizzled A as a regulator of healing processes after myocardiac infarction. By overexpressing Frizzled A in transgenic mice they demonstrate that cardiac function is improved. Although this report shows a correlation between overexpression of Frizzled A and a decrease of cytosolic β-catenin after myocardiac infarction, the authors are not able to directly trace back the improvement of cardiac function to the decrease of cytosolic β-catenin. The only acceptable conclusion from this study is that blocking WNT-signaling by Frizzled A results in reduced infarct size and cardiac rupture. It is not possible to make any statements about the role of β-catenin as many different factors upstream of β-catenin are involved in WNT-signaling and may contribute to the observed effect. Furthermore WNT-dependent signaling can involve other, alternative mediators (Liu et al. Cell 2002; 108:837-847). No link has been established to the role of β-catenin in enhancing endogenous cardiac regeneration.

In summary, the exact role of β-catenin in adult cardiac remodeling *in vivo* is still not understood.

Coming from this state of the art, it is an object of the present invention to provide an alternative method and means for the specific treatment of cardiovascular diseases or disorders.

Furthermore it is an object of the present invention to provide a heart medication for the specific treatment of cardiovascular diseases or disorders.

The objects of the invention are solved by the features of the independent claims.

### Summary of the invention

The present invention is directed to the use of agents capable of downregulating β-catenin dependent signaling for the treatment of cardiovascular diseases or disorders. As shown in the examples and figures, the specific inhibition of β-catenin dependent signaling by downregulating or inhibiting the expression or activity of β-catenin results in adaptive cardiac hypertrophy that is protective for example after angiotensin-II-induced stress.

In a preferred embodiment of the present invention the cardiovascular diseases are selected from the group of heart failure syndromes on the basis of chronic hypertension, ischemia, cardiac infarction, myocarditis and genetic causes. For those skilled in the art it is obvious that any other disease or disorder related to cardial cells or tissues are also within the scope of the present invention, comprising vascular or cellular damage as the basis for heart failure. Explicitly such diseases include those of the pericardium, heart valves, myocardium, blood vessels and veins. Furthermore a person skilled in the art knows that the present invention is applicable for mammals, especially the mammal being human.

It is intended according to the present invention that the agent is an inhibitor of β-catenin expression or activity or a modulator downregulating β-catenin expression or activity.

In further aspects the inhibitor or modulator is an expression construct that is transferred into the cardiac tissue or cells. A preferred method for delivering the expression construct into the cell is transfection, wherein known substances for alleviating transfer of the expression construct through the cell membrane are within the scope of the present invention.

Genetic material comprising nucleic acids, polynucleotides, RNA and DNA, of either natural or synthetic origin, including recombinant RNA and DNA and antisense RNA and DNA; hammerhead RNA, ribozymes, antigene nucleic acids, both single and double stranded RNA and DNA and analogs thereof, either in combination or not with other elements such as, for example, without limitation, cardiac tissue or cell specific enhancers, and nuclear localization signals, can be introduced into eukaryotic cells or organisms/patients via transformation or transfection techniques. The present invention uses an "expression construct", "nucleic acid construct" or alternatively a "nucleotide construct" or alternatively a "DNA construct". The term "construct" is used herein to describe a molecule, such as a polynucleotide may optionally be chemically bonded to one or more additional molecular moieties, such as a vector, or parts of a vector. In a specific--but by no means limiting--aspect, a nucleotide construct is exemplified by a DNA expression constructs suitable for the transformation of a cardiac host cell. As used herein, the terms "transformation" and "transfection" are intended to refer to a variety of art-recognized techniques for introducing foreign nucleic acid into a host cell/or tissue, including, for example, without limitation, calcium phosphate or calcium chloride co-precipitation, DEAE-dextran-mediated transfection, li-pofection, or electroporation. Suitable methods for transforming or transfecting host cells can be found in Sambrook, et al. (supra), and other laboratory manuals. For stable transfection of mammalian cells, it is known that, depending upon the expression vector and transfection technique used, only a small fraction of cells may integrate the foreign DNA into their genome.

In order to obtain an efficient in vivo transfer of the bioactive therapeutic agent of the present invention, various transfection agents are employed. Representative examples of transfection agents which are suitable for use with the methods of the present invention include, without limitation, calcium phosphate or calcium chloride co-precipitation, DEAE-dextran-mediated transfection, quaternary ammonium amphiphile DOTMA ((dioleoyloxypropyl)trimethylammonium bromide). Lipophilic glutamate diesters with pendent trimethylammonium heads; the metabolizable parent lipids such as-the cationic lipid dioctadecylamido glycylspermine (DOGS) and dipalmitoyl-phosphatidyl ethanolamylspermine (DPPES); metabolizable quaternary ammonium salts (DOTB, N-(1-[2,3-dioleoyloxy]propyl)-N,N,N-trimethylammonium methylsulfate (DOTAP), polyethyleneimine (PEI), dioleoyl esters, ChoTB, ChoSC, DOSC); 3beta[N-(N',N'-dimethylaminoethane)-carbamoyl]cholesterol (DC-Chol), dioleoylphosphatidyl ethanolamine (DOPE)/3beta[N-(N',N'-dimethylaminoethane)-carbamoyl]cholesterolDC-Chol in one to one mixtures, spermine, spermidine, lipopolyamines, lipophilic polylysines (LPLL), [[(1,1,3,3-tetramethylbutyl)cresoxy]ethoxy]ethyl]dimethylbenzylamionium hydroxide (DEBDA hydroxide) with excess phosphatidylcholine/cholesterol, cetyltrimethylammonium bromide (CTAB)/DOPE mixtures, lipophilic diester of glutamic acid (TMAG) with DOPE, CTAB, DEBDA, didodecylammonium bromide (DDAB), and stearylamine in admixture with phosphatidylethanolamine, DDAB/DOPE (TransfectACE, GIBCO BRL), and oligogalactose bearing lipids. Also encompassed within the present invention is the use of various transfection enhancer agents to increase the efficiency of transfer of the bioactive therapeutic factor into cells. Suitable transfection enhancer agents include, for example, without limitation, DEAE-dextran, polybrene, lysosome-disruptive peptide, chondroitan-based proteoglycans, sulfated proteoglycans, polyethylenimine, polylysine, integrin-binding peptide CYGGRGDTP, linear dextran nonasaccharide, glycerol, cholesteryl groups tethered at the 3'-terminal internucleoside link of an oligonucleotide, lysophosphatide, lysophosphatidylcholine, lysophosphatidylethanolamine, and 1-oleoyl lysophosphatidylcholine.

The intended expression constructs according to the present invention encode in a preferred embodiment for proteins inhibiting or downregulating β-catenin expression or activity. The interference with β-catenin expression or activity through the encoded proteins may be mediated by direct interaction or indirectly.

Preferred proteins to be encoded by the expression construct for the use according to the invention are Axin, Axin2, Chibby, Dapper, Human naked cuticle, Frizzled A, Dickkopf, Nemo-like kinase or Inhibitor of β-catenin (ICAT).

Further it is an objective of the present invention to select proteins as agent to be used that specifically target serin/threonin residues of β-catenin necessary for GSK3-β phosphorylation inducing subsequent degradation of β-catenin, more specific they are all localized in the N-terminal end of β-catenin coded by exon3, namely serine 33, serine 37, threonin 41 and serine 45. For a person skilled in the art it is obvious that kinases enhancing phosphorylation of these sites are also preferred proteins that are encoded by the expression constructs for the use according to the invention as they will lead to reduced cellular β-catenin levels.

As it is known for those skilled in the art that the COOH-terminal end of the "inhibitor of β-catenin and TCF" (ICAT) binds to the groove formed by armadillo repeats 5 to 9 of β-catenin, this site is known to be crucial for the binding to the transcription factor TCF and E-cadherin. Any other proteins blocking this site will also inhibit β-catenin dependent transcription and are therefore potentially useful in enhancing cardiac repair in heart failure. Especially casein kinase 1 (CK1) also enhances β-catenin degradation and is therefore a potentially useful therapeutic agent in cardiovascular disease.

In further aspects upstream inhibitors of the WNT/β-catenin pathway like members of the group of secreted frizzled-related proteins (sFRP) like FrizzledA (FrzA) and other inhibitory Wnt signals are also preferred proteins to negatively influence expression or activity of β-catenin. FrzA was shown to prevent cardiac rupture after experimental myocardiac infarct in mice (L. Barandon et al., Circulation 2003; 108:2282-89) by enhancing capillary density and reducing infarct size. Other upstream inhibitors being potentially useful in cardiac regeneration are the following proteins and their homologs: dickkopf-1 (Dkk), Frizzled B, nemo-like kinase (NLK), proteins enhancing NLK activity like "krüppel like factor - 15 (KLF15)", inhibitors of bcl9, activators of βTrCP.

It is self understood for a person skilled in the art, that inhibition or downregualtiion of β-catenin expression or activity can be achieved by cellular expression of the expression constructs and the encoded proteins on one hand and on the other hand by delivery of soluble forms of the above mentioned proteins specifically targeted to cardiac cells or tissues as an alternative for delivery of the proteins. For specific delivery of the agent to cardiac cells or tissues it is also an option to encapsulate the agent in a carrier targeted to cardiac cells or tissues. Additionally it is intended, that the carrier can be "opened" by external measures like supersonic pulse resulting in release of the encapsulated agent. This measures are also preferred within therapeutic use of the agent to achieve release at a defined time point during therapy. For a person skilled in the art it is obvious that a carrier can contain more than one agent and subsequent release of the agents can be achieved.

To assure cardiac-specific expression of the used expression constructs of β-catenin inhibitors (direct on indirect) promoters like the α-myosin heavy chain (αMHC) and myosin light chain 2c (MLC2v) promoter are preferred, as it is likely that unspecific inhibition of β-catenin in various cells or tissues not belonging to the heart or vessels related to the supply of cardiac cells or tissues will cause severe side-effects. The used expression control elements are also intended to be inducible, for instance by hormones or antibiotics.

Furthermore expression constructs are intended which are targeted to β-catenin expression controlling elements, wherein the expression controlling elements are deoxyribonucleic acid sequences controlling β-catenin gene transcription, like promotors or other β-catenin specific gene transcription control elements.

Preferably the used expression constructs are plasmids. It is also intended that the expression constructs represent an adenoviral vector, an adenoassociated vector, a retroviral vector or a lentiviral vector.

The term "vector" refers to a nucleic acid molecule capable of transporting another nucleic acid to which it has been linked. The term "expression vector" or "expression construct" includes any vector, (e.g., a plasmid, cosmid, phage or artificial chromosome) containing a gene construct in a form suitable for expression by a cell (e.g., linked to a promoter). In the present specification, "plasmid" and "vector" are used interchangeably, as a plasmid is a commonly used form of vector. Moreover, the invention is intended to include other vectors which serve equivalent functions.

Also contemplated within the present invention is the use of a virus-like particle containing a bioactive therapeutic agent according to the invention, wherein the virus-like particle is physically linked to the transfection agent, which is also linked to the microparticle. Such virus-like particles may be designed using polyethylenimine (PEI) conjugated to a therapeutic sequence. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids (vectors). However, the invention is intended to include such other forms of expression vectors, such as viral vectors (e.g., replication defective retroviruses, adenoviruses and adeno-associated viruses), which serve equivalent functions.

Another aspect is the use of an expression construct for in vivo gene targeting, wherein the in vivo gene targeting is preferably mediated through the expression of small interference RNA targeted to β-catenin RNA. One recently detected way of inhibiting gene expression is based on the production of double-stranded RNA molecules. Using such double-stranded RNA (dsRNA), targeted switching off of single genes is possible in a highly effective manner and more rapidly compared to any other method, without impeding protein formation of neighboring genes. The basic principle is referred to as RNA interference, abbreviated as RNAi, and the dsRNA sequence responsible for this phenomenon as siRNA (small interference RNA).

The siRNA does not prevent reading of the gene, but rather switches on a cellular mechanism causing degradation of the mRNAs read from the gene, thus preventing formation of the corresponding protein (post-transcriptional gene silencing).

Another embodiment of the present invention is the use of an expression construct being covalently linked to signaling elements specific for cardiac cells or tissue. As already mentioned the scope of the present invention is restricted to the inhibition of β-catenin expression or activity in cardiac cells or tissues. To assure specific delivery of expression constructs it is intended to use cardiac cell or tissue specific signaling elements.

Standard techniques for the construction of expression vectors suitable for use in the present invention are well-known to those of ordinary skill in the art and can be found in such publications as Sambrook et al. (1989) Molecular Cloning: A Laboratory Manual, 2nd Ed. Cold Spring Harbor, N.Y. A variety of strategies are available for ligating fragments of DNA, the choice of which depends on the nature of the termini of the DNA fragments and which choices can be readily made by the skilled artisan.

The recombinant expression vectors of the invention can also be designed for expression of a polypeptide in eukaryotic cells (e.g., insect cells (using baculovirus expression vectors), yeast cells, or mammalian cells, or in an organism), whereby these cells are usable as drugs or pharmaceutical compositions in gene therapy. Suitable host cells are discussed further in Goeddel, supra.

In a preferred embodiment, a nucleic acid is expressed in mammalian cells or an organism using a mammalian expression vector. Examples of mammalian expression vectors include pCDM8 and pMT2PC. When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook et al., supra.

In another embodiment, the recombinant mammalian expression vector is capable of directing expression of the nucleic acid preferentially in cardiac cells or tissues. Tissue-specific regulatory elements are known in the art.

The term "gene delivery" or "gene transfer" refers to methods or systems for reliably inserting foreign DNA into target cells, such as into muscle cells or others. Such methods can result in transient or long term expression of genes. Gene transfer provides a unique approach for the treatment of acquired and inherited diseases. A number of systems have been developed for gene transfer into mammalian cells.

The amount of viral vector in the composition may vary according to factors such as the disease state, age, sex, and weight of the individual. Dosage regimens may be adjusted to provide the optimum therapeutic response. For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian or non-mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of individuals.

In addition to virus-mediated gene-delivery systems, there are several nonviral options for gene delivery. The simplest method is the direct introduction of therapeutic DNA into target cells. This approach is limited in its application because it can be used only with certain tissues and requires large amounts of DNA.

Another non-viral approach involves the creation of an artificial lipid sphere with an aqueous core. This liposome, which carries the therapeutic DNA, is capable of passing the DNA through the target cell's membrane.

Therapeutic DNA also can get inside target cells by chemically linking the DNA to a molecule that will bind to special cell receptors. Once bound to these receptors, the therapeutic DNA constructs are engulfed by the cell membrane and passed into the interior of the target cell. This delivery system tends to be less effective than other options.

Persons skilled in the art are also experimenting with introducing a 47^{th} (artificial human) chromosome into target cells. This chromosome would exist autonomously alongside the standard 46 - not affecting their workings or causing any mutations. It would be a large vector capable of carrying substantial amounts of genetic code, and scientists anticipate that, because of its construction and autonomy, the body's immune systems would not attack it. A problem with this potential method is the difficulty in delivering such a large molecule to the nucleus of a target cell.

Another aspect of the present invention are known inhibitors of β-catenin and their use for downregulation or inhibition of β-catenin expresision or activity in cardiac cells or tissues. Explicitly the use of 13-cis-retinoic-acid, indometacin, PKF 118-310, Celecoxib, SKI-606 and Quercitin are intended.

In further aspects the agent is selected from the group of proteins, peptides, antibodies, nucleic acids or small molecules.

The invention also relates to a pharmaceutical composition, comprising the gene therapy construct of the invention and a pharmaceutically acceptable carrier.

Where clinical applications are contemplated, it will be necessary to prepare pharmaceutical compositions in a form appropriate for the intended application. Generally, this will entail preparing compositions that are essentially free of pyrogens, as well as other impurities that could be harmful to humans or animals.

One will generally desire to employ appropriate salts and buffers to render delivery vectors stable and allow for uptake by target cells. Buffers also will be employed when recombinant cells are introduced into a patient. Aqueous compositions of the present invention in an effective amount may be dissolved or dispersed in a pharmaceutically acceptable carrier or aqueous medium. Such compositions also are referred to as inocula. The phrase pharmaceutically or pharmacologically acceptable refers to molecular entities and compositions that do not produce adverse, allergic, or other untoward reactions when administered to an animal or a human. As used herein, pharmaceutically acceptable carrier, includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the vectors or cells of the present invention, its use in therapeutic compositions is contemplated. Supplementary active ingredients also can be incorporated into the compositions.

As used herein, the terms "pharmaceutically acceptable", "physiologically tolerable" and grammatical variations thereof, as they refer to compositions, carriers, diluents and reagents, are used interchangeably and represent that the materials are capable of administration to or upon a human without the production of undesirable physiological effects such as nausea, dizziness, gastric upset and the like which would be to a degree that would prohibit administration of the composition.

The preparation of a pharmacological composition that contains an agent for the use according to the invention dissolved or dispersed therein is well understood in the art. Typically such compositions are prepared as sterile injectables either as liquid solutions or suspensions, aqueous or non-aqueous, however, solid forms suitable for solution, or suspensions, in liquid prior to use can also be prepared. The preparation can also be emulsified.

The active agent can be mixed with excipients which are pharmaceutically acceptable and compatible with the active ingredient and in amounts suitable for use in the therapeutic methods described herein. Suitable excipients are, for example, water, dextrose, glycerol, ethanol or the like and combinations thereof. In addition, if desired, the composition can contain minor amounts of auxiliary substances such as wetting or emulsifying agents, as well as pH buffering agents and the like which enhance the effectiveness of the active ingredient.

The therapeutic pharmaceutical composition that contains an agent for the use according to the invention can include pharmaceutically acceptable salts of the components therein. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like.

Solutions of the active compounds as free base or pharmacologically acceptable salts can be prepared in water suitably mixed with a surfactant, such as hydroxypropylcellulose. Dispersions also can be prepared in glycerol, liquid polyethylene glycols, and mixtures thereof and in oils. Under ordinary conditions of storage and use, these preparations contain a preservative to prevent the growth of microorganisms.

The above-mentioned physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, propylene glycol, polyethylene glycol and other solutes.

Liquid compositions can also contain liquid phases in addition to and to the exclusion of water. Exemplary of such additional liquid phases are glycerin, vegetable oils such as cottonseed oil, organic esters such as ethyl oleate, and water-oil emulsions.

In addition to the active substance(s), i.e., the agent for the use according to the invention, solutions and emulsions may include conventional carriers such as solvents, solubilizers, and emulsifiers such as water, ethyl alcohol, isopropyl alcohol, ethyl carbonate, ethyl acetate, benzyl alcohol, benzyl benzoate, propylene glycol, 1,3-butylene glycol, dimethylformamide, oils, especially cotton seed oil, peanut oil, corn oil, olive oil, castor oil and sesame oil, glycerol, glycerol formal, tetrahydrofurfuryl alcohol, polyethylene glycols, and fatty esters of sorbitan, or mixtures of these substances. For parenteral application, the solutions and emulsions may also be present in a sterile and blood-isotonic form.

In addition to the active substance(s), suspensions may include conventional carriers such as liquid diluents, e. g. water, ethyl alcohol, propylene glycol, suspending agents, e. g. ethoxylated isostearyl alcohols, polyoxyehtylenesorbitol and sorbitan esters, microcrystalline cellulose, aluminum metahydroxide, bentonite, agar, and tragacanth, or mixtures of these substances.

Other examples for delivery of the agents for the use according to the present invention that may be employed include intra-arterial, intracavity, intravesical, intrathecal, intrapleural, and intraperitoneal routes.

Intra-arterial administration is achieved using a catheter that is inserted into an artery to an organ or to an extremity. Typically, a pump is attached to the catheter. Agents can be given directly via catheter.

A further aspect of the present invention relates to gene therapy and applications related to the prophylaxis, diagnosis, therapy, follow-up and/or aftercare of cardiac diseases.

The use of an agent that inhibits or downregulates β-catenin signaling or β-catenin expression or activity for the preparation of a remedy for the treatment of cardiovascular diseases or disorders is a further objective for the present invention.

Another aspect of the present invention is a heart medication comprising an agent capable of downregulating β-catenin dependent signaling in cardiac cells and/or tissues. Further the heart medication comprises an agent that is an inhibitor of β-catenin expression or modulator capable of downregulating β-catenin expression.

The agents contained as part of the heart medication according to the invention relate to all the agents mentioned above with the properties described in detail above.

Finally a method for the treatment of cardiaovascular diseases by application of an agent for downregulating and/or inhibiting β-catenin expression or activity in cardiac cells or tissues is an objective of the present invention. This method comprises direct injection of the agent in the heart.

### Detailed description of the invention

By studying the role of β-catenin in adult heart homeostasis and LV remodeling after chronic GPCR-stimulation it was found that decreased β-catenin expression caused modest cardiac hypertrophy at baseline, compared to controls. AngII infusion induced cardiac hypertrophy both in control - and in β-catenin depleted mice, but not in β-catenin -stabilized mice. This decreased hypertrophic response was accompanied by reduced fractional shortening. The phenotype of deteriorating heart function upon β-catenin stabilization was associated with altered regulation of the T-box proteins Tbx5 and Tbx20 as well as the AKT-regulated, atrophy-related protein IGF-binding protein 5 (IGFBP5). It can be concluded, that β-catenin downregulation is required for adaptive cardiac hypertrophy.

The two complementary mouse models analyzed in the examples below were generated employing precedingly described β-catenin floxed transgenic mice. No apparent phenotype linked to the membrane function of β-catenin in complex with N-cadherin was observed. The compensatory upregulation of plakoglobin (γ-catenin) demonstrated by Zhou et al. (Am J Physiol Heart Circ Physiol. 2007;38:120) in heart tissue might account for this phenomenon. This hypothesis is also supported by data from other tissues employing this very same floxed mouse strain (Huelsken et al. Cell. 2001;105:533-545; Zhou et al. Am J Physiol Heart Circ Physiol. 2007;38:120).

Similar considerations concerning the relevance of membrane-associated β-catenin versus nuclear β-catenin apply to the mouse model with β-catenin stabilization: while no apparent phenotype concerning rhythm disturbances or membrane dysfunction was observed, classical β-catenin target genes Axin2, Tcf-4 and Lef-1 were found upregulated in cardiac tissue extracts. The nuclear localization of the stabilized truncated β-catenin mutant in β-cat^{exon3 flox/flox} also accounts for other phenoytpes including the recently described block in haematopoetic stem cell differentiation (Harada et al. EMBO J. 1999;18:5931-5942; Scheller et al. Nat Immunol. 2006;7:1037-1047).

It was surprising to find that by stabilizing β-catenin in the adult heart, the hypertrophic response after AngII stimulation was abrogated; however at the price of deteriorating heart function. This observation contradicts earlier findings, where β-catenin depletion blocked cardiac hypertrophy after trans-aortic constriction (TAC) (Haq et al. Proc Natl Acad Sci U S A. 2003;100:4610-4615; Chen et al. Mol. Cell. Biol. 2006;26:4462-4473).

Several differences between the studies may explain the discrepant findings: The AngII stimulus employed here activates signaling cascades different from pressure overload as in TAC. Discrepant results concerning the same signaling molecule have been described before i.e. concerning the role of MEKK1 in cardiac hypertrophy (Minamino et al. Proc Natl Acad Sci U S A. 2002;99:3866-3871; Sadoshima Jet al. J. Clin. Invest. 2002;110:271-279). In β-catenin depleted mice, Chen et al. describe an increase in heart weight and upregulation of hypertrophy markers ANP and β-MHC when compared to baseline levels indicating intact hypertrophy in β-catenin depleted mice. The definition of hypertrophy was based on heart weight and cardiomyocyte width of isolated cardiomyocytes; neither diastolic wall size or cell surface area in tissue sections were reported (Chen et al. Mol. Cell. Biol. 2006;26:4462-4473). Intriguingly concerning the point of adaptive vs. mal-adaptive cardiac remodeling, β-catenin depleted mice preserved LV-function despite ongoing stress by TAC supporting a concept of adaptive hypertrophy mediated by β-catenin downregulation.

Another major difference between the studies is the time course of the experiments: as described below, mice were stimulated with AngII 10 days after mifepristone-induced Cre recombinase activation and analyzed 2 weeks later; Chen et al. exposed their mice to TAC 6 weeks after induction of Cre recombinase and analyzed them 2 weeks later. The initial delay after β-catenin depletion might have induced secondary effects (Chen et al. Mol. Cell. Biol. 2006;26:4462-4473).

The complimentary studies shown below employing mice with β-catenin stabilization seemingly confirm a beneficial role of β-catenin downregulation in cardiac hypertrophy: after AngII stimulation, the β-catenin -stabilized mice exhibited a deterioration of cardiac function. To the best of our knowledge, *in vivo* studies describing the phenotype of mice with genetic stabilization of β-catenin have not been described precedingly.

First, it has been tested whether cardiac β-catenin manipulation might affect cardiomyocyte apoptosis. While an increase of TUNEL/α-sarcomeric actin/DAPI positive nuclei after AngII treatment in control mice and β-catenin depleted mice to levels reported before was observed, this was not observed in the mice with β-catenin stabilization (van Empel et al. Circ Res. 2005;96:e92-e101). While determination of absolute values of apoptosis would require the combination of several techniques, these data clearly exclude increased apoptosis to account for the observed phenotype. This observation is in line with preceding findings: several studies analyzing tissue-specific alterations of β-catenin levels found that β-catenin regulated expression of survival genes in brain, thymocytes, and vascular smooth muscle cells (Wang et al. Circ Res. 2002;90:340-347). Precedingly the association of stabilized β-catenin and cell survival of isolated rat cardiomyocytes after activation of the PI3-kinase/AKT pathway has been described (Bergmann et al. Journal of Molecular and Cellular Cardiology. 2004;37:681-690).

Next, it has been analyzed precedingly described target genes of β-catenin and PI3kinase/AKT. The PI3-kinase/AKT pathway was precedingly shown to control β-catenin levels in cardiomyocytes (Haq et al. Proc Natl Acad Sci U S A. 2003;100:4610-4615). As β-catenin downregulation initiates heart formation in the embryo, the gene expression levels of different transcription factors related to cardiomyocyte differentiation were investigated. Interestingly, differential regulation of T-box proteins Tbx5 and Tbx20 was found: AngII treatment downregulated Tbx5 mRNA and protein expression in control mice, whereas this effect was attenuated in β-cat^{Δex3} mice. Tbx20 was upregulated in mice with β-catenin stabilization. Differential roles have been identified for these T-box proteins: Tbx5 was precedingly shown to promote cardiomyocyte differentiation in association with Nkx2.5 (Hiroi et al. Nat Genet. 2001;28:276-280). In addition, heterozygote Tbx 5 deletion is a model for the Holt-Oran syndrome; interestingly, by 8 weeks after birth such mice develop a phenotype of diastolic dysfunction known to precede systolic dysfunction (Zhou et al. Am J Physiol Heart Circ Physiol. 2005;289:H992-1001). In contrast, Tbx20 is known to exert multiple transcriptional repressive functions including inhibition of ANP expression (Stennard et al. Development. 2005;132:2451-2462; Plageman and Yutzey. J. Biol. Chem. 2004;279:19026-19034). Increased expression of Tbx20 might therefore inhibit cardiac growth in β-cat^{Δex3} mice.

In addition, regulation of the IGF-binding protein 5 (IGFBP5) was observed. In general, IGFBP5 inhibits cell proliferation and differentiation but increases cell survival rates (Schneider et al. J Endocrinol. 2002;172:423-440). Upregulation of IGFBP5 has been detected in mouse models with chronic activation of AKT (Matsui et al. J Biol Chem. 2002;277:22896-22901). Prolonged activation of this pathway was recently found to induce a phenotype of dilated cardiomyopathy (Shiojima et al. J. Clin. Invest. 2005;115:2108-2118; Nagoshi et al. J. Clin. Invest. 2005;115:2128-2138). The known functions of IGFBP5 and its expression levels observed here make this protein another candidate for explaining the effect of β-catenin stabilization on cardiac left ventricular remodeling.

In summary, downregulation of β-catenin initiates adaptive cardiomyocyte hypertrophy in the adult heart. Stabilizing β-catenin in the adult heart blocks signaling pathways required for protective hypertrophy after AngII-induced stress. This effect was not associated with detectable changes in intercellular adhesion but with altered gene expression concerning Tbx5 and Tbx20, which belong to the cardiac embryonic gene program. This finding suggests that upon hypertrophic stress, the adult heart reverts to regulation of β-catenin in a fashion similar to β-catenin involvement in embyronic cardiac development.

The invention is further illustrated by the following examples and figures which should not be construed as limiting:

### Examples

### Example 1: The β-catenin heart-specific mouse models

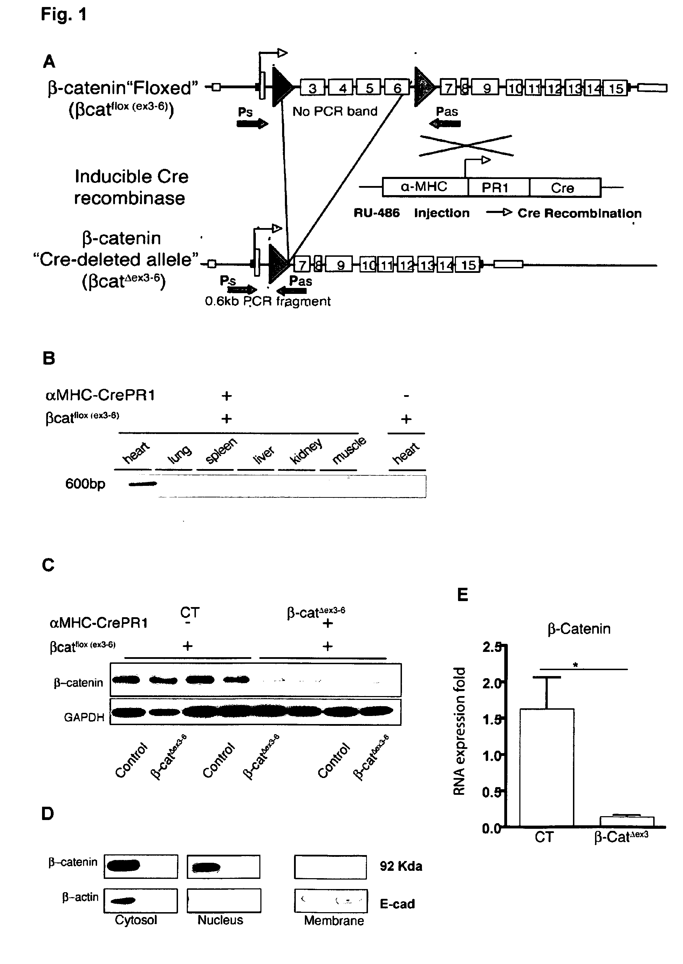

An inducible Cre/loxP system restricting β-catenin depletion to α-MHC positive cells after hormone induction was employed. Recombination efficiency was tested through mating of α-MHC-CrePR1 mice to ROSA 26 reporter mice expressing the lacZ expression cassette under the control of a floxed stop codon. Recombination efficiency after mifepristone injection was determined to be approx. 70% of cardiomyocytes. Induction of Cre recombinase excised exons 3-6 in hearts from β-cat^{ex3-6 flox/flox} x αMHC-CrePR1, resulting in animals that had a heart-specific β-catenin depletion (β-cat^{Δex 3-6}, Fig. 1B). Successful heart-specific recombination was confirmed by PCR using primers for the recombined DNA that result in a 600 bp fragment (Fig. 1B). No product was obtained from lung, spleen, liver, kidney, or skeletal muscle (Fig. 1B). The down-regulation of β-catenin was detected both at the mRNA (Fig. 1E) and protein level analyzing total heart extracts from several mice (Fig. 1C): Active Cre recombinase led to dramatically reduced β-catenin mRNA and protein (Fig. 1C, D and E). Biochemical methods showed a depletion of β-catenin in all cellular compartments including the nucleus in whole heart tissue extracts. Employing antibodies against both the N- and C-terminus, expression of a truncated β-catenin isoform was excluded as described before (Fig. 1C and D). In tissue, β-catenin was readily detectable in gap junctions between cardiomyocytes in control hearts, but not in sections from β-catenin heart-depleted mice. Staining of N-cadherin complexing with β-catenin at the cell junction showed no apparent phenotype in β-cat^{Δex3-6} mice with this method (data not shown). Nuclei were stained with DAPI. β-catenin staining was clearly reduced in the mutants while N-cadherin was not affected. Furthermore an enlargement of an area containing a gap junction, which shows the apparent depletion of β-catenin expression was detectable. Electrocardiograms showed no rhythm disturbances in the mice (data not shown).

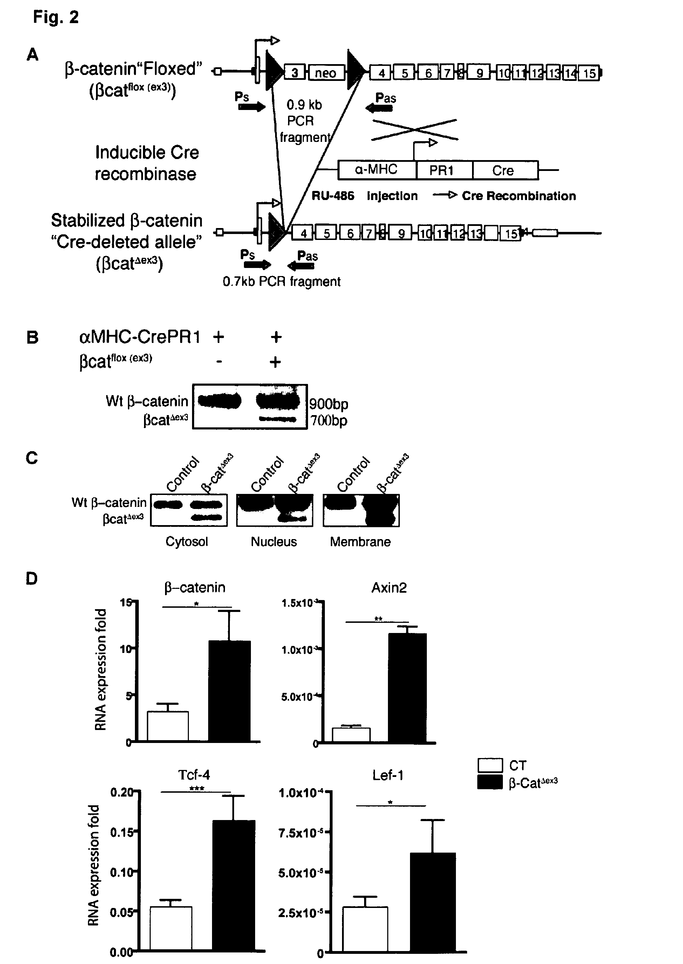

Deletion of exon 3 renders β-catenin to be resistant against GSK3β-induced phosphorylation and consequently blocks proteasom-mediated degradation. Mifepristone administration to β-cat^{ex3 flox/flox} mice mated to α-MHC-CrePR1 mice generated β-cat^{Δex3} animals (Fig. 2A). Heart-specific Cre recombinase activation excised exon 3 and resulted in mice with heart-restricted, stabilized β-catenin (Fig. 2A). PCR using primers designed to give a 700 bp fragment after successful recombination in contrast to 900 bp beforehand confirmed the genotype (Fig. 2B). Stabilized β-catenin was detectable in cytosolic, membrane and nuclear fractions of whole heart lysates as a smaller band than endogenous β-catenin (Fig. 2C). Staining with N-cadherin, which is complexing with β-catenin at the cell junction, did not reveal an apparent phenotype at the membrane compared to control mice (data not shown). Next, genes regulated transcriptionally by β-catenin were analyzed by real-time PCR with extracts from β-cat^{Δex3} mutants compared to control littermates: Axin2 (7.03±0.7 fold, p< 0.001), Tcf-4 (2.92±0.16 fold, p< 0.05) and Lef-1 (2.91±0.4 fold, p<0.05) expression was significantly increased (Fig. 2 D). These data prove a functional increase in β-catenin dependent transcription in β-cat^{Δex3} mice as described before concerning other tissues.

### Example 2: β-catenin depletion leads to mild cardiac hypertrophy

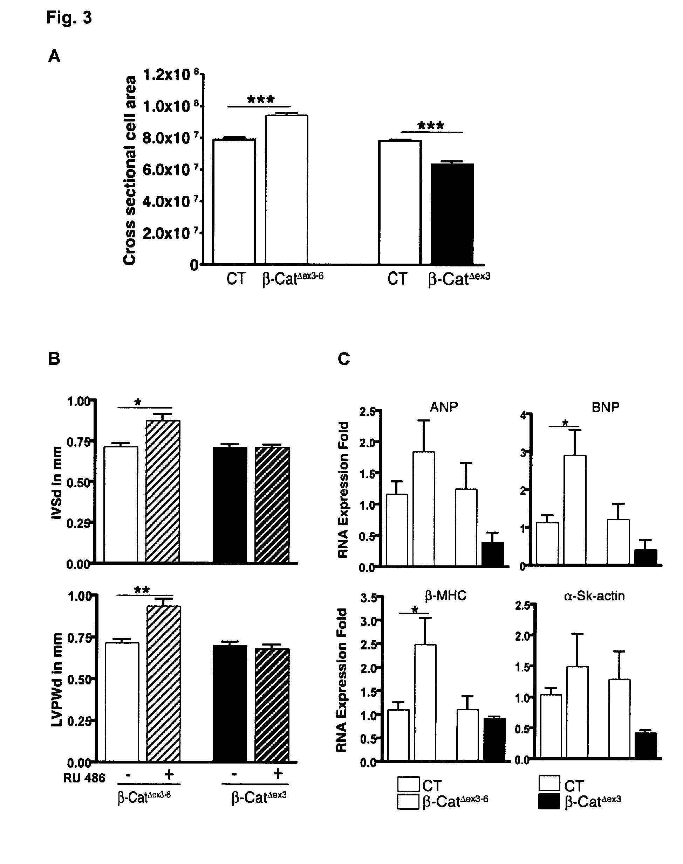

Fourteen days after the final mifepristone injection, the cardiac transverse ventricular cross-sections appeared thicker in β-catenin depleted, compared to wild-type mice (data not shown). Cardiac hypertrophy was confirmed by cross sectional area analysis *in vivo*: the average cell size from β-catenin depleted mice (9.4x10⁷ ± 0.14x10⁷) increased significantly over control cell size (7.9x10⁷ ± 0.15x10⁷, P<0.001) (Fig. 3A). Echocardiographic examination demonstrated increased diameters of the diastolic interventricular septum as well as the left ventricular posterior wall (Fig. 3B). No significant changes were seen regarding cavity size or fractional shortening. A summary of all echocardiographic measurements can be found in supplementary table 1. RNA quantification revealed increased expression of hypertrophy gene markers atrial and brain natriuretic peptides (ANP, BNP), β myosin heavy chain (β-MHC), and α-sarcomeric actin (Fig. 3C). In summary, depletion of β-catenin leads to moderate cardiac hypertrophy in the adult heart. This initial phenotype did not exaggerate and was not accompanied by a loss of cardiac function or any other cardiac deterioration up to the age of 6 months (data not shown).

### Example 3: β-catenin stabilization leads to impaired cardiac growth

Deletion of the endogenous exon 3 results in a single copy gene of stabilized β-catenin under the control of the endogenous reporter. In contrast to our initial hypothesis based on precedingly published *in vitro* and *in vivo* data ^{12, 13,} β-catenin stabilization (β-cat^{Δex3}) did not cause hypertrophy (Fig. 3). In fact, when cross-sectional area was calculated 14 days after Cre recombinase induction by mifepristone, a significantly decreased cell size was observed (CT 7.8x10⁷ ± 1.1x10⁶ vs β-cat^{Δex3}6.4x10⁷ ± 1.4x10⁶ p<0.001, Fig. 3A). Cells from stabilized β-catenin mice were approximately 82% the size of control cells. Hypertrophy gene marker expression was the inverse of the β-catenin depletion experiment. Hypertrophic markers were either stable (β-MHC) or downregulated in the β-cat^{Δex3} samples compared to controls (Fig. 3C). Over six months, the animals developed and behaved normally. Taken together, the above data suggest that depleting β-catenin in the adult heart results in adaptive cardiac hypertrophy, while expression of a non-degradable mutant results in a phenotype of smaller cardiomyocytes indicating impaired cardiac growth or cardiac muscular atrophy.

### Example 4: Responses to AngII in the β-catenin mouse models

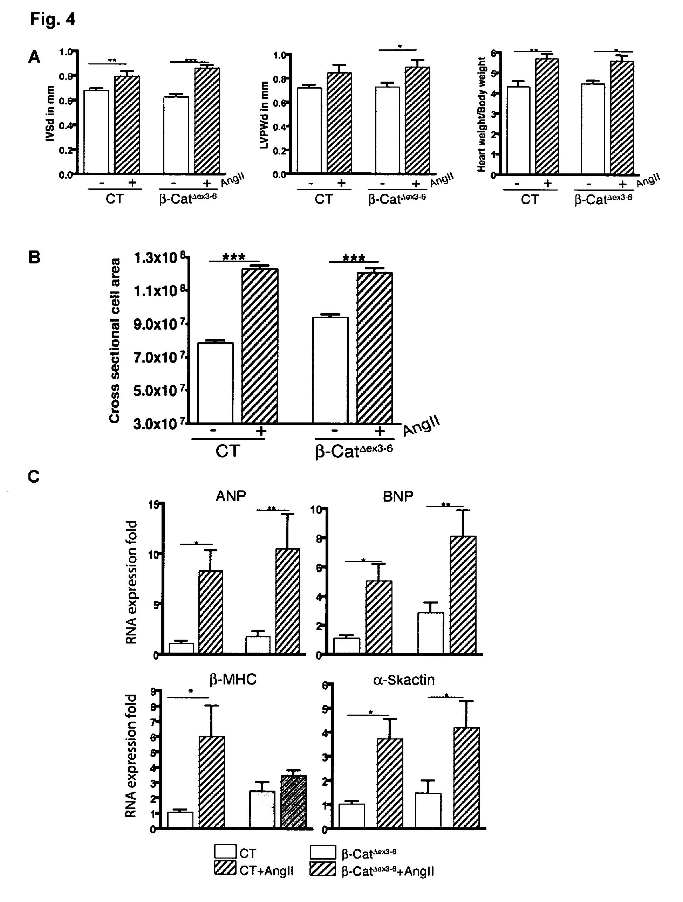

After 12 days' AngII infusion at pressure doses (1.4 µg Kg⁻¹ min⁻¹), control animals developed hypertrophy as judged by echocardiography, gene regulation, *in vivo* cross sectional area, and heart/body weight ratio as described before (Fig 4).¹⁰ From the M-mode echocardiography imaging, a thickened inter-ventricular septum and LV posterior wall after AngII was identified in control animals (Fig. 4 A). Even if at baseline β-catenin depleted mice exhibited an increase of the cardiomycyte area (data not shown), treatment with AngII further enhanced cellular cross sectional area (12.1x10⁷ ± 0.24x10⁷ a.u.) to absolute values similar to control mice (12.3x10⁷ ± 0.19x10⁷ a.u.) (Fig. 4B). LV wall thickening was identical in β-catenin heart-depleted animals and control littermates after AngII (data not shown). Heart weights normalized to body weight showed a significant increase for both control and β-catenin depletion mice (Fig. 4A, suppl. table 1). Moreover, AngII increased gene expression of several hypertrophy gene markers in both control and b-catenin heart-depleted animals with the exception of β-myosin heavy chain (Fig. 4 C). A lack of β-MHC upregulation in response to stress stimuli might be indicative of adaptive cardiac remodeling preserving LV-function as described before (van Rooij E et al., Circ Res. 2004;94:18e-26).

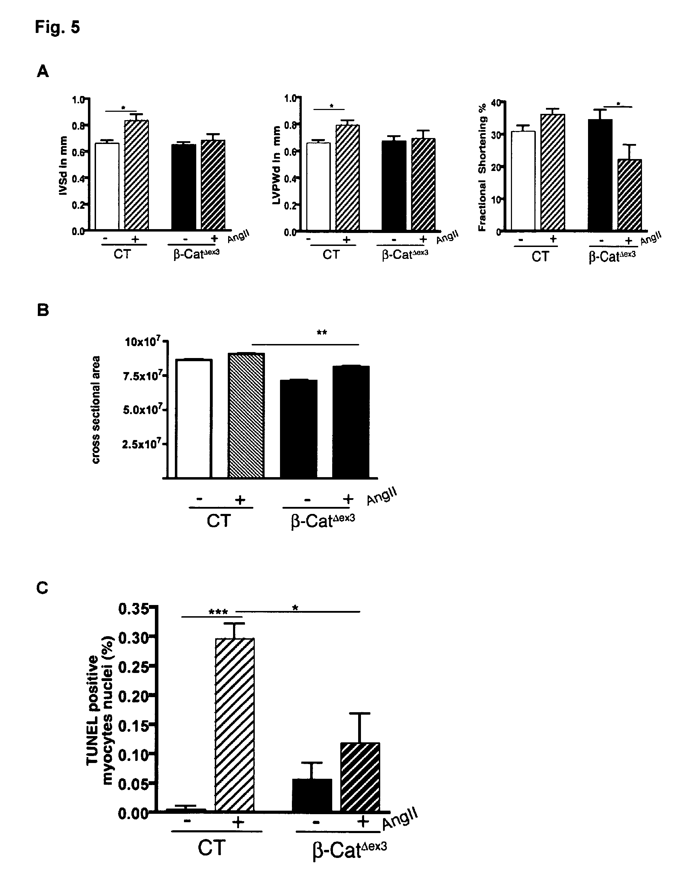

The effects of 12 days' AngII infusion in mice with cardiac stabilized β-catenin (β-cat^{Δex3}) were opposite in kind to those of mice with β-catenin heart-depletion. Echocardiography showed no significant change in the wall thickness following AngII treatment in β-catenin stabilized mice in contrast to control littermates, where wall thickness increased significantly (data not shown). ANP (β-cat^{Δex3} 0.4 ± 0.15; β-cat^{Δex3}+AngII 1.03 ± 0.15; p< 0.01) and BNP (β-Cat^{Δex3} 0.43 ± 0.24; β-cat^{Δex3}+AngII 1.4 ± 0.27; p< 0.01) expression was induced by AngII but final values were generally lower than in control mice (ANP: CT + AngII 2.3 ± 0.8; BNP: CT + AngII 2.6 ± 0.6). *In vivo,* cardiomyocyte size following AngII treatment significantly increased over baseline in both models, but was significantly smaller in β-cat^{Δex3} compared to control animals treated with AngII (CT 9.07x10⁷ ± 0.14x10⁷ vs β-cat^{Δex3} 8.14x10⁷ ± 0.14x10⁷, P<0.001; Fig. 5B).

As expected, fractional shortening increased in control mice after AngII treatment (31.1±1.6% to 36.2±1.6%). Instead, β-catenin heart-stabilization led to reduced fractional shortening after AngII infusion (34.7±2.9% to 22.4±4.5%, p< 0.05). These absolute values in animals aged 10 to 14 weeks are identical to a serial analysis of the age-dependant changes employing the same high-resolution echo techniques as used for these studies. The reduced fractional shortening in β-catenin stabilized mice was not due to increased cardiomyocyte apoptosis as β-catenin stabilized mice demonstrated rather fewer TUNEL/α-sarcomeric actin/DAPI-positive cells after AngII treatment compared to controls (β-cat^{Δex3}: 0.058±0.027% at baseline to 0.120±0.049% after AngII; p < 0.001 compared to CT + AngII 0.297+0.025%; Fig. 5C). In contrast, analyis of mice with β-catenin depletion showed a non-significant increase of TUNEL -positive cells at baseline with similar results after AngII treatment (CT: 0.004±0.004; β-cat^{Δex 3-6}: 0.112±0.044%; after AngII: CT 0.09±0.02%; β-Cat^{Δex3}-6: 0.2±0.13%). These absolute values are similar to other reports, i.e. rates of apoptotic cardiomyocytes after trans-aortic constriction. ¹⁶ Moreover, no significant differences concerning fibrosis occurring focally throughout the LV wall was observed comparing both transgenic models to their respective controls. In summary, AngII induced hypertrophy is not affected by β-catenin depletion, while mice with stabilized β-catenin exhibit an impaired hypertrophic response to AngII. Apoptosis-related mechanisms do not explain these findings.

### Example 5: Analysis of cardiac β-catenin target genes

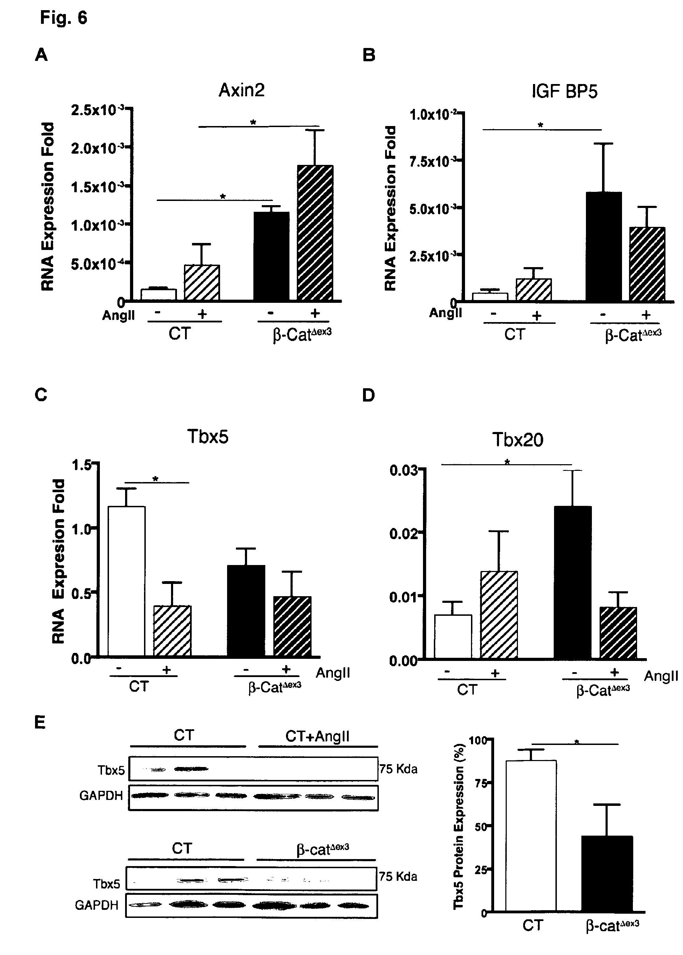

Next, several target genes were analyzed precedingly described to be regulated in a β-catenin - dependent fashion either in embryonic development (GATA-4, Nkx 2.5, MEF2a, Tbx5 and Tbx20) or have been described in the context of chronic PI3-kinase/AKT signaling (atrogin 1, IGFBP5) (Skurk et al. J. Biol. Chem. 2005;280:20814-20823). Analysis was performed in whole heart RNA extracts. GATA-4, Nkx2.5, MEF2a and atrogin 1 expression was not significantly altered by AngII treatment or α-MHC-dependent changes in β-catenin levels. Axin2 levels were increased by AngII in both control and β-catenin stabilized mice, although the baseline levels were already significantly increased in β-cat^{Δex3} mice (Fig. 6A, Fig. 2D).

Interestingly, β-catenin levels affected gene expression of IGFBP5, a protein inhibitory for the IGF-signaling cascade. IGFBP5 was found upregulated in hearts with genetically mediated chronic AKT activation (Skurk et al. J. Biol. Chem. 2005;280:20814-20823). Functionally, IGFBP5 is implicated in muscle atrophy. Here, IGFBP5 was upregulated in β-cat^{Δex3} mice compared to control littermates (9.02±3.2 fold). No significant change of expression levels were observed upon AngII stimulation.

At baseline, β-catenin stabilization led to reduced Tbx5 mRNA and protein levels while Tbx20 mRNA was found to be upregulated (Fig. 6C+D; Tbx20 vs. control: 3.3±0.31 fold, p< 0.05). AngII treatment significantly downregulated Tbx5 gene expression in control mice; this effect was lost in the β-catenin stabilized mice (Fig. 6 C). No significant effect of AngII on Tbx20 gene expression was observed (Fig. 6C). These results suggest differential regulation of T-box proteins downstream of β-catenin in association with a phenotype of impaired cardiac growth and performance.

### Description of the figures

- **Fig. 1**: Generation of mice with inducible, heart specific β-catenin depletion (β-cat^{Δex3-6})
- **Fig. 2**: Generation of mice with inducible, heart specific stabilization of β-catenin (β-cat^{Δex3})
- **Fig. 3**: Baseline characterization reveals modest hypertrophy in β-cat^{Δex3-6}and diminished growth in β-cat^{Δex3}
- **Fig.4**: AngII stimulation induces hypertrophy in both control and β-catenin depleted mice
- **Fig.5**: β-catenin stabilization attenuates AngII-induced cardac hypertrophy but leads to decreased cardiac function
- **Fig. 6**: Gene regulation in β-cat^{Δex3} mice

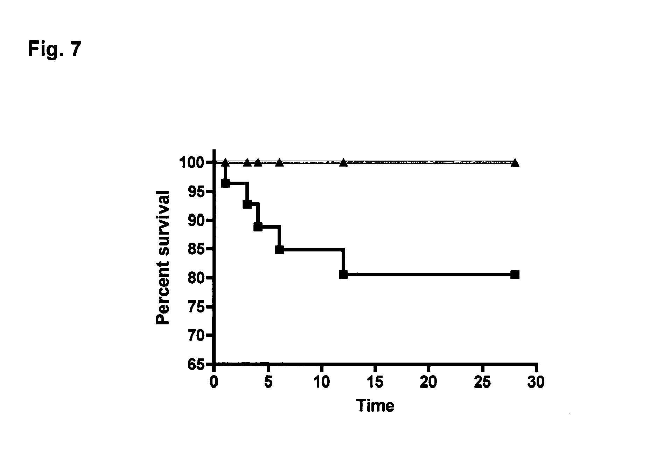

- **Fig. 7**: Kaplan-Meier survival analysis

### Figure 1

(A) Representative mating scheme between mice carrying β-catenin floxed exons 3-6 and αMHC-CrePR1 mice. Mice have been described precedingly. For heart-specific, progesterone inducible Cre recombinase expression αMHC-CrePR1 mice were employed. In β-cat^{ex3-6 flox/flox} x α-MHC-CrePR1, administration of mifepristone leads to loxP site recombination with the appearance of a 600bp band (B). Hybridization sites of the primers are indicated *(red arrows).* (C) Western blot (WB) from whole heart lysate of both control and β-cat^{Δex3-6} mice. A significant reduction of β-catenin protein expression in β-cat^{Δex3-6} is detected by a C-terminal binding antibody. (D) A second WB employing a N-terminal binding antibody indicates diminished expression of β-catenin in all cellular compartments (cytosol, nucleus and membrane). β-actin was used as control for cytosol and nucleus and E-cadherin for membrane. (E) Also at the mRNA level, β-catenin quantification by Real Time PCR normalized to GAPDH revealed decreased expression of full length β-catenin cDNA *(right panel).*

### Figure 2

(A) The scheme depicts the mating between α-MHC-CrePR1 mice and β-cat^{Δx3 flox/flox}, which upon recombination lack the GSK 3β phosphorylation site resulting in organspecific stabilization of β-catenin. (B) Genomic PCR analysis shows the truncated β-catenin product (700bp) following specific recombination in the heart of in β-cat^{Δex3} mice; hybridization sites of the primers are indicated (*red arrows).* (C) Western blot depicting expression of the truncated form of β-catenin protein in β-Cat^{Δex3} mice in different cellular compartments (cytosol, nucleus and membrane). (D) β-catenin target genes Axin2, Tcf-4 and Lef-1 are upregulated in β-cat^{Δex3} total heart RNA extracts as measured by real time PCR. Fold expression is relative to GAPDH calculated as copy numbers.

### Figure 3

A quantification of transverse sections of control, β-cat^{Δex3-6} and β-cat^{Δex3} hearts is shown. Cross sectional area analysis by Wheat germ agglutinin (WGA)-FITC staining demonstrates modest cardiac hypertrophy in mice with cardiac-specific β-catenin depletion (β-cat^{Δex3}-6) as well as diminished growth after β-catenin stabilization (β-cat^{Δex3}). n=8-11 mice per group. (B) Echocardiographic diastolic diameters of inter-ventricular septum (IVSd) and left ventricular posterior wall (LVPWd) demonstrate cardiac hypertrophy after β-catenin depletion. No significant change in wall dimensions after β-catenin stabilization was observed (n≥15 for each group). (C) RNA quantification by real time RT-PCR analysis of hypertrophic markers after β-catenin depletion and stabilization. *=P<0.05, **=P<0.01; expression fold is relative to GAPDH control.

### Figure 4

(A) Both control and β-catenin depleted β-cat^{Δex3-6} mice exhibit cardiac hypertrophy after 14 days of microosmotic minipump infusion of AngII (1.4 µg/kg⁻¹/min⁻¹). Representative examples of M-mode echocardiograms (not shown), quantification of echocardiographic parameters and heart/body weight from control and β-cat^{Δex3-6} mice with and without AngII infusion are shown (n≥6 for each group) (B) Myocyte cross-sectional area increases in both control and β-catenin depleted β-cat^{Δex3-6} mice as measured by WGA-FITC staining of cardiac tissue sections and quantification from control or β-cat^{Δex3-6} mice with AngII infusion (data not shown). At least 500 cells were measured for each animal, n≥4 mice per group. (C) Significant upregulation of ANP, BNP and α-sarcomeric actin normalized to GAPDH after AngII in control and β-catenin depleted β-cat^{Δex3-6} mice. Quantification by real-time PCR of whole heart RNA samples from control (white) or β-cat^{Δex3-6} (grey) mice without (open bars) and with (shaded bars) AngII infusion. *=P<0.05, **=P<0.01, ***=P<0.001.

### Figure 5

(A) Attenuation of cardiac hypertrophy after AngII in β-catenin stabilized mice is accompanied by significant deterioration of systolic function as measured by fractional shortening (right bar graph). Representative examples of M-mode echocardiograms and quantification from control or β-cat^{Δex3} mice with and without AngII infusion. Quantification of echocardiographic wall size and fractional shortening from control or β-cat^{Δex3} mice with and without AngII infusion. *=P<0.05, **=P<0.01. n≥3. (B) Increase of myocyte cross-sectional area after AngII is attenuated in β-catenin stabilized β-cat^{Δex3} mice (data not shown). At least 400 cells counted per animal, n≥3 mice per group. ***=P<0.001. (C) Rates of TUNEL/α-sarcomeric actin/DAPI-positive cardiomyocytes after AngII infusion decrease after β-catenin stabilization in β-cat^{Δex3}. In addition to the TUNEL-assay, slides were co-stained with α-sarcomeric actin and DAPI in control mice and β-cat^{Δex3} mice before and after AngII stimulation (data not shown).

### Figure 6

RNA normalized to GAPDH levels in whole heart extracts as measured by Real Time PCR in β-catenin stabilized mice (β-cat^{Δex3}). (A) Up-regulation of β-catenin target gene Axin 2 by AngII treatment in β-cat^{Δex3} compared to control animals. (B) Upregulation of Insulin like - Growth Factor Binding Protein 5 (IGFBP5) was found by RT-PCR at baseline in the β-cat^{Δex3} mice in comparison to controls, while no significant change was observed by AngII stimulation. (C) T-box protein 5 (Tbx5) and (D) T-box protein 20 (Tbx20) mRNA levels are differentially regulated in β-cat^{Δex3} mice upon AngII stimulation. (E) Tbx5 Western blot showing the down-regulation of its expression in control mice after AngII treatment *(upper panel)* as well as in β-cat^{Δex3} mice at baseline *(lower* panel). WB quantification was done by densitometry normalized to GAPDH *(right* panel).

### Figure 7

A Kaplan-Meier survival analysis of control mice (line with squares; n = 11) and β-catenin depleted mice (line with triangles; n = 26) was performed over 30 days after myocardial infarct. The above described effect of β-catenin depletion is reflected by the higher survival rate of the β-cat^{Δex3-6} mice in comparison to the control mice. Consequently the invention claims downregulation or inhibition of β-catenin expression or activity for the treatment of cardiovascular diseases or disorders.

## Claims

1. The use of an agent for downregulating or inhibiting β-catenin dependent signaling for the treatment of cardiovascular diseases or disorders.

2. The use according to claim 1, wherein the cardiovascular diseases are selected from the group of heart failure syndromes on the basis of chronic hypertension, ischemia, cardiac infarction, myocarditis and genetic causes.

3. The use according to claim 1 or 2, wherein the agent is an inhibitor of β-catenin expression or downregulating modulator of β-catenin expression.

4. The use according to any of the preceding claims, wherein the agent is an inhibitor of β-catenin activity or downregulating modulator of β-catenin activity

5. The use according to any of the preceding claims, wherein the inhibitor or modulator is an expression construct.

6. The use according to claim 5, wherein the expression construct encodes for proteins inhibiting or downregulating β-catenin expression or activity.

7. The use according to claim 6, wherein the encoded protein is Axin, Axin2, Chibby, Dapper, Human naked cuticle, Frizzled A, Dickkopf, Nemo-like Ligase or Inhibitor of beta-catenin (ICAT).

8. The use according to claim 5, wherein the expression construct is targeted to β-catenin expression controlling elements.

9. The use according to claim 8, wherein the expression controlling elements are deoxyribonucleic acid sequences controlling β-catenin gene transcription.

10. The use according to any of the claims 5 to 9, wherein the expression construct is a plasmid.

11. The use according to any of the claims 5 to 9, wherein the expression construct is an adenoviral vector, an adenoassociated vector, a retroviral vector or a lentiviral vector.

12. The use according to any of the preceding claims, comprising an expression construct for in vivo gene targeting.

13. The use according to claim 12, wherein the in vivo gene targeting is mediated through the expression of small interference RNA targeted to β-catenin RNA.

14. The use according to any of the preceding claims 5 to 13, wherein the expression construct is covalently linked to signaling elements specific for cardiac cells or tissue.

15. The use according to any of the preceding claims, wherein the expression construct comprises expression control elements specific for cardiac cells or tissues.

16. The use according to claim 15, wherein the expression control elements are inducible.

17. The use according to any of the preceding claims 1 to 4, wherein the agent is 13-cis-retinoic-acid.

18. The use according to any of the preceding claims 1 to 4, wherein the agent is indometacin.

19. The use according to any of the preceding claims 1 to 4, wherein the agent is PKF 118-310.

20. The use according to any of the preceding claims 1 to 4, wherein the agent is Celecoxib.

21. The use according to any of the preceding claims 1 to 4, wherein the agent is SKI-606.

22. The use according to any of the preceding claims 1 to 4, wherein the agent is Quercitin.

23. The use according to any of the claims 1 to 4, wherein the agent is selected from the group of proteins, peptides, antibodies, nucleic acids or small molecules.

24. The use according to any of the preceding claims, wherein the agent is encapsulated in a carrier targeted to cardiac cells or tissues.

25. The use according to claim 24, wherein the carrier releases the agent after supersonic pulse.

26. The use according to any of the preceding claims, **characterized in that** a pharmaceutically acceptable carrier is comprised.

27. The use according to any of the preceding claims, **characterized in that** the carrier is selected from the group comprising fillers, disintegrants, binders, humectants, extenders, dissolution retarders, absorption enhancers, wetting agents, adsorbents and/or lubricants.

28. The use according to any of the preceding claims, **characterized in that** agent is a capsule, a tablet, a coated tablet, a suppository, an ointment, a cream, an injection solution and/or an infusion solution.

29. The use according to any of the preceding claims, **characterized in that** said agent is an oral, vaginal, rectal, nasal, topical, subcutaneous, intravenous, intramuscular, intraperitoneal composition, suppository, pad and/or foam.

30. The use according to any of the preceding claims for gene therapy and applications related to the prophylaxis, diagnosis, therapy, follow-up and/or aftercare of cardiac diseases.

31. Use of an agent that inhibits or downregulates β-catenin signaling for the preparation of a remedy for the treatment of cardiovascular diseases or disorders.

32. The use according to claim 31, wherein the expression or activity of β-catenin is specifically downregulated or inhibited.

33. Heart medication comprising an agent for downregulating or inhibiting β-catenin dependent signaling in cardiac cells and/or tissues.

34. Heart medication according to claim 33, **characterized by** an agent that is an inhibitor of β-catenin expression or modulator capable of downregulating β-catenin expression.

35. Heart medication according to claims 33 or 34, **characterized by** an agent that is an inhibitor of β-catenin activity or modulator capable of downregulating β-catenin activity

36. Heart medication according to any of the preceding claims 33 to 35, wherein the inhibitor or modulator is an expression construct.

37. Heart medication according to claim 36, wherein the expression construct encodes for proteins interfering with β-catenin expression or activity.

38. Heart medication according to claim 36, wherein the expression construct is targeted to β-catenin expression controlling elements.

39. Heart medication according to claim 38, wherein the expression controlling elements are deoxyribonucleic acid sequences controlling β-catenin gene transcription.

40. Heart medication according to any of the preceding claims 36 to 39, wherein the expression construct is a plasmid.

41. Heart medication according to any of the preceding claims 36 to 39, wherein the expression construct is an adenoviral vector, an adenoassociated vector, a retroviral vector or a lentiviral vector.

42. Heart medication according to any of the preceding claims 33 to 41, comprising an expression construct for in vivo gene targeting.

43. Heart medication according to claim 42, mediating the in vivo gene targeting through the expression of small interference RNA targeted to β-catenin RNA.

44. Heart medication according to any of the preceding claims 36 to 43, wherein the expression construct is covalently linked to signaling elements for cardiac cells or tissue.

45. Heart medication according to any of the preceding claims 33 to 35, wherein the agent is 13-cis-retinoic-acid.

46. Heart medication according to any of the preceding claims 33 to 35, wherein the agent is indometacin.

47. Heart medication according to any of the preceding claims 33 to 35, wherein the agent is PKF 118-310.

48. Heart medication according to any of the claims 33 to 35, wherein the agent is selected from the group of proteins, peptides, antibodies, nucleic acids or small molecules.

49. Heart medication according to any of the preceding claims 33 to 48, **characterized in that** a pharmaceutically acceptable carrier is comprised.

50. Heart medication according to any the preceding claims 33 to 49, **characterized in that** the carrier is selected from the group comprising fillers, disintegrants, binders, humectants, extenders, dissolution retarders, absorption enhancers, wetting agents, adsorbents and/or lubricants.

51. Heart medication according to any of the preceding claims 33 to 50, **characterized in that** agent is a capsule, a tablet, a coated tablet, a suppository, an ointment, a cream, an injection solution and/or an infusion solution.

52. Heart medication according to any of the preceding claims 33 to 51, **characterized in that** said agent is an oral, vaginal, rectal, nasal, topical, subcutaneous, intravenous, intramuscular, intraperitoneal composition, suppository, pad and/or foam.

53. Heart medication according to any of the preceding claims 33 to 51 for gene therapy and applications related to the prophylaxis, diagnosis, therapy, follow-up and/or aftercare of cardiac diseases.

54. A method for the treatment of cardiaovascular diseases by application of an agent for downregulating and/or inhibiting β-catenin expression or activity in cardiac cells or tissues.

55. A method according to claim 27, wherein the agent is injected directly in the heart.
